Europäisches Patentamt

European Patent Office

Office européen des brevets

(19)

(11) Publication number: **0 134 758**
**A2**

(12) # EUROPEAN PATENT APPLICATION

(21) Application number: **84830120.6**

(22) Date of filing: **17.04.84**

(51) Int. Cl.⁴: **A 61 M 5/14**

(30) Priority: **18.04.83 IT 4812983**

(43) Date of publication of application:
**20.03.85 Bulletin 85/12**

(84) Designated Contracting States:
**CH DE FR GB LI**

(71) Applicant: **Bombardieri, Giuseppe**
**Strada Poggino 5**
**I-01100 Viterbo(IT)**

(72) Inventor: **Bombardieri, Giuseppe**
**Strada Poggino 5**
**I-01100 Viterbo(IT)**

(54) Device for the controlled insulin or glucose infusion in diabetic subjects.

(57) A device is described consisting of an implantable semipermeable hollow-fiber circuit (1), through which a saline solution (3) is pumped (2), a sensor (4) for the glucose assay on the said solution and a microcomputer unit (6) which controls an insulin (7) and a glucose (8) infusion pump on the basis of glucose concentration measurements. The hollow-fibers form a filter into which low molecular weight molecules may diffuse until the equilibrium is reached with their concentration in the surrounding body fluid.

EP 0 134 758 A2

./...

Croydon Printing Company Ltd.

DEVICE FOR THE AUTOMATIC INSULIN OR GLUCOSE INFUSION IN DIABETIC
SUBJECTS, BASED ON THE CONTINUOUS MONITORING OF THE PATIENT'S
GLUCOSE, OBTAINED WITHOUT BLOOD WITHDRAWAL.

TITLE MODIFIED
see front page

## Background of the invention

This invention refers to a new device in the field of the feedback controlled (or "closed-loop") insulin pumps, known also as
"artificial pancreas". These devices provide a continuous glucose
determination in the diabetic patient; data are transmitted from
a glucose sensor to a microprocessor unit, which controls a pump
for insulin (or glucose) infusion, in order to maintain blood
glucose levels within a physiological range. Glucose determination in the actually known closed-loop insulin pumps is achieved
by two different methods. In the first one blood is continuously
withdrawn from a patient's vein and then reaches a glucose sensor
(directly or after ultrafiltration). The main disadvantages of
the system are the blood loss and/or the blood clotting in the
drawing tubes. The second method to obtain a continuous glucose
assay consists of the sensor insertion into the patient's body,
usually in the subcutaneous tissue. The drawback of the system is
the rapid loss of sensor liability, probably due to fibrin and
some blood cells which unavoidably reach the sensor tip. The
above mentioned disadvantages of the known closed-loop insulin
pumps allow only a short-term use of the device.
In the system according to the present invention the samples for
a continuous glucose monitoring in the diabetic patient are
obtained from a saline solution that is pumped through a hollow-
fiber circuit implanted in the patient's body. Hollow-fibers are
permeable to small molecules: thus glucose diffuses into the
fiber lumen from the surrounding body fluid. When equilibrium is reached, the pumped solution is ready for glucose assay by a suitable glucose sensor. This method avoids blood withdrawal (with the related clotting problems) and provides a long-term reliable glucose determination in diabetic patients. My invention is of special interest for the realization of a "wearable" or implantable closed-loop insulin pump.

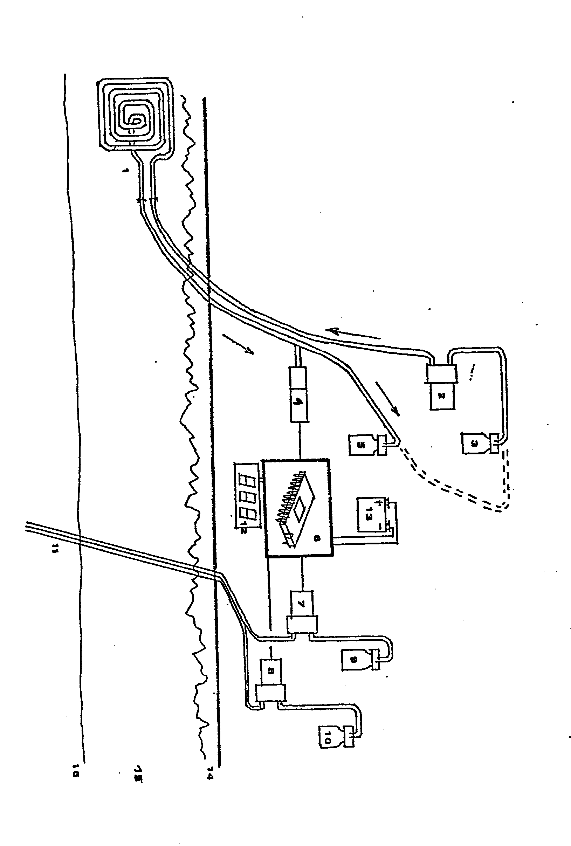

The figure is a schematic diagram of the device.

Description of the device

The device consists of four main parts:
a) the hollow-fiber saline solution circuit;
b) the glucose sensor;
c) the electronics;
d) the infusion pumps.

a) the hollow-fiber saline solution circuit.

This part is totally novel. A saline solution is pumped from a reservoir (3) to the glucose sensor (4) through a hollow-fiber circuit (1) by means of a piezoelectric or peristaltic pump (2). The hollow-fibers are made of plastic biocompatible material and are permeable only to small molecules. Plastic biocompatible materials (such as polyacrylonytrile or polyethylene with a cutoff of 40000 normally used in dyalisis filters) may be used. The hollow-fiber circuit must be implanted in the patient's body, usually in the subcutaneous tissue (15), through a short incision of the skin (14) using any local anaesthetic. The hollow-fiber fragility and the need of an adequate circuit lenght in the minimum space may require special arrangements of the fiber with suitable supports. The figure shows a spiral-shaped hollow-fiber circuit (1) sustained by a small plastic disk (not shown), implanted in the subcutaneous tissue of the abdominal wall (15). The hollow-fiber number and lenght are chosen so as to obtain, in a short time, a uniform concentration of glucose in the solution inside the fiber lumen and in the surrounding body fluid. Biocompatible plastic tubes connect the implanted circuit with the pump (2) and with the glucose sensor (4). After the glucose assay the solution flows towards a waste reservoir (5) or can be repumped to the hollow-fiber circuit (dashed-line in the figure).

### b) Glucose sensor

The glucose sensor (4) described in this system is an enzymatic-potentiometric one, commonly used in many labóratory instruments for blood glucose determination and also in the commercially available artificial pancreas, such as for instance, BIOSTATOR, marketed by MILES Laboratories, USA.

### c) The electronics

The amplified signal coming from the glucose sensor (4) enters a microprocessor unit (6) by means of an analog to digital converter. The microprocessor stores the data and, on the basis of a suitable algorithm (Albisser, Diabetes, May 74, p.396), controls the insulin and the glucose infusion pumps. It also shows in the display (12) the data coming from the glucose sensor and the amounts of the infused insulin and glucose. The microprocessor may be for instance the Motorola model MC1468705R3 a CMOS 8 bit MCU with the timer, a four-channel A/D converter, 2.8 Kbyte EPROM, 128 byte RAM, four I/O ports inside. The power supply of the all system is provided by suitable batteries (13).

## d) The infusion pumps

Insulin (7) and glucose (8) pumps are controlled by the microprocessor. Solenoid or piezoelectric micropumps can be used to infuse insulin and glucose with high precision. Insulin and glucose are taken from special reservoirs (9,10) and can be infused into the patient by separate biocompatible catheters or by a single dual lumen catheter (11). The administration route can be the abdominal cavity (16), the subcutaneous tissue or a periferal vein.

The device according to the present invention has been tested in animals (rabbits and guinea pigs) without any loss of sensivity because the hollow-fiber circuit does not become clogged up with blood or fibrin and thus the components of blood and fibrin do not reach the sensor. The glucose measurements made with the device according to the present invention have been compared with glucose measurements made with a conventional laboratory apparatus, specifically the instrument Yellow Springs YSI Model 23A and the values agree, showing that the device gives a reliable determination. The device may be adapted to a size of 8x10x3 cm. and may be implanted into the abdominal wall of the patient. Thus the present invention permits to avoid the insertion of the sensor directly into the subcutaneous tissue of a patient and, at the time, it permits to avoid withdrawal of blood. The device according to the present invention may be used not only in the case of diabetic patients for the determination of glucose, but also for the determination of many low molecular weight components in body fluids, such as sodium, potassium, calcium etc., and also medicines (for instance digitalis) administered to humans, so that their concentration may be monitored.

WHAT I CLAIM IS

1) A device consisting of a permeable to the small molecules hollow-fiber circuit, which is implanted into animals or humans, and through which a pumped solution reaches the same small molecules concentration as in the surrounding body fluid; the said solution is used for the assay of parameters of biological or medical interest.

2) The device according to claim 1) wherein the pumped solution is used for glucose concentration measurement in order to realize a body glucose concentration monitor or an artificial pancreas.

3) The device according to claim 1) wherein the pumped solution is used for the measurement of sodium, potassium, calcium, or administered drugs.